# EUROPEAN PATENT APPLICATION

(11) **EP 4 491 216 A1**
(43) Date of publication of application: **15.01.2025**
(21) Application number: 23184913.4
(22) Date of filing: 12.07.2023
(51) Int. Cl.: A61N 1/375

(54) **SPRING CONTACT IN PERMANENT IMPLANTS, PARTICULARLY HEADERS**

(71) Applicant: BIOTRONIK SE & Co. KG, 12359 Berlin (DE)
(72) Inventor: Litzke, Jan, 13507 Berlin (DE)
(74) Representative: Biotronik Corporate Services SE

(57) **Abstract**

The present invention relates to a medical device (1), comprising: a first component (10, 101, 115) comprising an electrically conducting contact surface (10a), a second component (11, 101, 111, 115) having an electrically conducting contact member (11a), and an electrically conducting connection device (2) electrically connecting the contact surface (10a) of the first component (10) to the contact member (11) of the second component (11). According to the present invention, the connection device (2) comprises a base portion (20) electrically connected to said contact member (11), a contact portion (22), and at least one spring (21) electrically connecting the base portion (20) to the contact portion (22), wherein the at least one spring (21) is tensioned and thereby presses a front side (22a) of the contact portion (22) against the contact surface (10a) so that the contact portion (22) permanently contacts the contact surface (10a).

## Description

The present invention relates to a medical device, particularly an implantable medical device.

Typically, soldered or welded joints are used in medical devices, particularly implantable medical devices, to assure a reliable electrical contact between electrical components of such a device.

However, welded joints require not only expensive system technology (often comprising lasers) for a proper alignment of components to be connected, but also a high degree of precision during assembly. In addition, conditions relating to occupational health and safety are necessary. Since welded joints are rigid, there is always a risk that movement in the area of the weld can cause the transfer forces to break the joint. Similar considerations apply to soldered joints. At the same time, separate internal wiring strips have to be designed and procured in small quantities for each product.

Based on the above, the problem to be solved by the present invention is to avoid an overdetermination of welded joints and their assembly processes, particularly to provide an electrical contact means for a medical device that offers suitable compensation of tolerances of components that are to be connected.

This problem is solved by a medical device having the features of claim 1. Preferred embodiments are stated in the corresponding dependent clams and are described below.

According to claim 1, a medical device is disclosed, comprising:
- a first component comprising an electrically conducting contact surface,
- a second component having an electrically conducting contact member, and
- an electrically conducting connection device electrically connecting the contact surface of the first component to the contact member of the second component.

According to the present invention, the connection device comprises a base portion electrically connected to said contact member, wherein the connection device further comprises a contact portion, and at least one spring electrically connecting the base portion to the contact portion, wherein the at least one spring is tensioned and thereby presses a front side of the contact portion against the contact surface of the first component so that the front side of the contact portion permanently contacts the contact surface of the first component.

Thus, the spring-loaded contact portion may replace existing welded joints e.g., by spring contacting an internal structure of a permanent implant. However, such contacting may also be applied in the external area of a header of an implantable medical device.

According to an embodiment, the at least one spring, the base portion, the contact portion or the whole connection device according to the invention comprise a core portion made from stainless steel, particularly MP-35N, or beryllium copper, titanium copper, phosphor bronze or brass. In one embodiment, the core portion comprises a first coating on a first surface, particularly an outer surface, wherein the coating comprises or essentially consists of gold and/or tin. In one embodiment, the core portion comprises a second coating an a second surface, particularly an inner surface, wherein the second coating comprises or essentially consists of nickel.

According to an embodiment of the present invention, the contact surface of the first component is a contact pad. However, it is also conceivable - see further below - that the contact surface can be formed by a plug (e.g., of an electrode lead) that is contacted by the spring-loaded contact portion (e.g., in a socket of a header of the medical device).

Particularly, spring contacting according to the present invention may be used in the internal structure of permanent implants such as implantable cardiac pacemakers, implantable cardioverter defibrillators, implantable cardiac monitors, neurostimulators, vagus nerve stimulator, and even intracardiac pacemakers (e.g., implantable leadless pacemakers). Advantageously, the invention particularly allows one to carry out the assembly of the components without internal wiring strips as well as welded joints.

Furthermore, according to an embodiment of the present invention, the at least one spring is wound around a virtual central axis extending orthogonal to the front side of the contact portion, wherein the contact portion faces the base portion in a direction defined by the central axis. Further, in preferred embodiment of the present invention, the spring is compressed in said direction and thereby tensioned. In one embodiment, the at least one spring comprises an insulating coating, particularly on an outside of the wound spring. In one embodiment, the connection device according to the invention comprises two springs each wound around a virtual central axis extending orthogonal to the front side of the contact portion, wherein the contact portion faces the base portion in a direction defined by the central axis, wherein particularly each of the springs comprises an insulating coating, particularly such that both springs are partly insulated from each other, particularly at a portion of each of the springs extending between the base portion and the contact portion of the connection device according the invention.

According to a further embodiment of the present invention, the at least one spring integrally connects the contact portion to the base portion.

Furthermore, according to an embodiment of the present invention, the base portion is connected to the contact member by one of: a welded connection, a soldered connection.an electrically conductive adhesive connection, a crimp connection

Preferably, in an embodiment, the front side of the contact portion comprises a planar region contacting the contact surface, wherein the planar region preferably comprises an area in the range from 0.01 mm² to 1.5 mm², preferably 0.1 mm² to 1 mm², most preferably 0.1 mm² to 0.5 mm². Particularly, such planar region of the contact area guarantees that the electrical connection provided by the connection device can handle relatively high electrical currents, e.g., up to 2000 V at 30 A.

According to yet another embodiment, the front side comprises a contact nose for contacting the contact surface, wherein the contact nose is preferably elastically supported on the contact portion so that the contact nose is flush with a surrounding region of the front side (particularly with the planar region), when the front side is pressed against the contact surface by the at least one spring.

Furthermore, according to a further embodiment, the connection device comprises a second spring for pressing the front side of the contact portion against the contact surface.

Preferably, in an embodiment, the second spring connects (particularly integrally) the contact portion to the base portion. Alternatively, the second spring butts against a back side of the contact portion and is connected to the base portion, wherein said back side faces away from said front side. Here, the second spring can tension the contact portion by pressing against it, but without being actually connected to the contact portion.

According to a further embodiment, said second spring is wound around said virtual central axis, too.

Further, according to an embodiment of the invention, the first component is a battery, and wherein the second component is a capacitor or vice versa.

Further, according to an embodiment of the invention, the first component is a feedthrough and wherein the second component is an electronic module or vice versa.

Further, according to an embodiment of the invention, the first component is an electrode lead and the contact surface is formed by a plug of the electrode lead, and wherein the second component is a header (e.g., of an implantable medical device such as a cardiac pacemaker or the like) comprising a socket for receiving said plug.

Furthermore, according to an embodiment of the present invention, the medical device is an implantable medical device. Preferably, the implantable medical device is one of: an implantable cardiac pacemaker, an implantable cardioverter defibrillator, an implantable cardiac monitor (i.e., a device configured for recording and transmitting physiological data relating to the patient carrying the cardiac monitor), an implantable leadless pacemaker (i.e., an intracardiac pacemaker, where all electrodes are arranged on a housing of the pacemaker and are not formed as leads), a neurostimulator, a vagus nerve stimulator.

However, according to yet preferred embodiment, the medical device may also be a medical device configured for use outside the human or animal body, wherein preferably the medical device is one of: an external pacemaker, a blood sugar sensor, a blood pressure measuring device, an external neurostimulator, an external cardiac monitor, a battery, a capacitor, a cancer monitoring device.

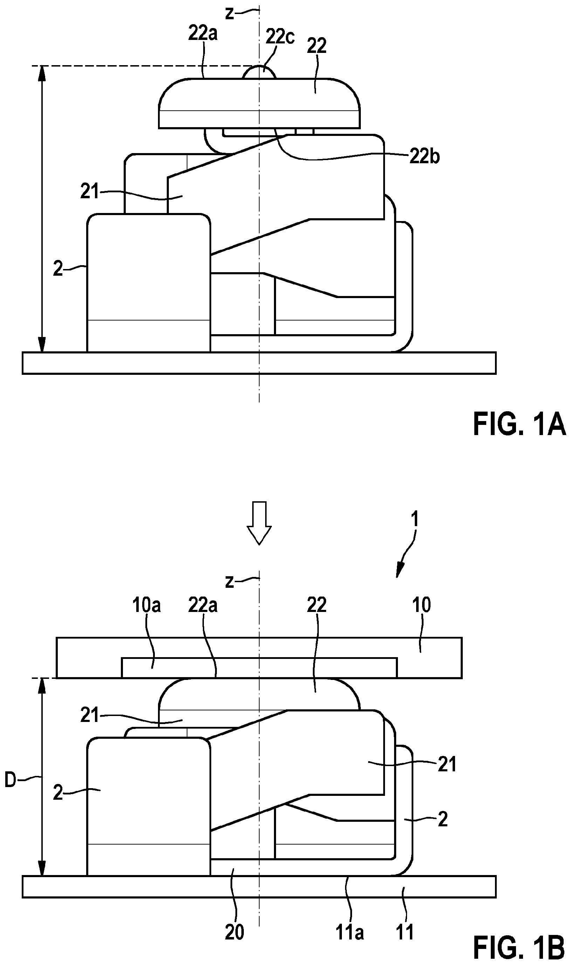

In the following, embodiments as well as further features and advantages of the present invention shall be described with reference to the Figures, wherein
- Fig. 1: shows an embodiment of a medical device according the present invention using a connection device configured to press a contact portion against a contact surface using at least one spring, wherein (A) shows the relaxed spring and (B) shows the spring being tensioned, and

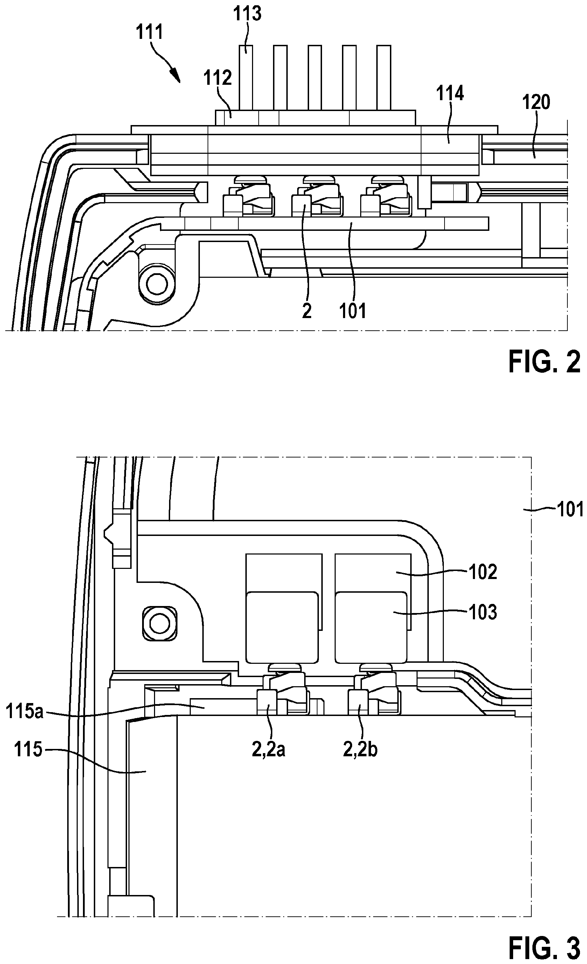

- Fig. 2: shows (A) a typical connection between electrical components using a flex connector as known in the prior art, and (B) an embodiment of the present invention wherein multiple electrically conducting connections between the electrical components are provided based on a corresponding number of connection devices.

Fig. 1 (A) and (B) show an embodiment of a medical device 1 according to the present invention which comprises a connection device 2 for electrically connecting two components 10, 11 of the medical device 1, here depicted in a schematical fashion (cf. Fig. 1 (B)).

Particularly, the medical device 1 comprises a first component 10 comprising an electrically conducting contact surface 10a, here e.g., in form of a contact pad, and a second component 11 having an electrically conducting contact member 11a (e.g. a pin, contact pad or the like) that is in electrical contact with an electrically conducting connection device 2 that electrically connects the contact surface 10a of the first component 10 to the contact member 11a of the second component 11, thus connecting the two components 10, 11 such that an electrical current may be passed between them. Particularly, the connection device 2 comprises a base portion 20 being electrically connected to said contact member 11a, a contact portion 22, and at least one spring 21 electrically connecting the base portion 20 to the contact portion 22, wherein the at least one spring 21 is tensioned and thereby presses a front side 22a of the contact portion 22 against the contact surface 10a of the first component 10 so that the contact portion 22 permanently contacts the contact surface 10a of the first component 10. Preferably, the front side 22 is or comprises a flat planar region comprising a sufficient area to allow also high currents to pass through the contact surface 10a and front side 22a of the contact device 2. Optionally, the front side 22a can comprise a contact nose 22c as exemplary shown in Fig. 1 (A), which contact nose 22c is preferably elastically supported on the contact portion 22 and can therefore by arranged flush with the front side / planar region 22a. Particularly, Fig. 1 (A) shows the contact device 2 before electrical contact is made to the contact surface 10a of the first component 10. Fig. 1 (B) shows the installed connection device 2, wherein the at least one spring 21 presses the contact portion 22 with the front side 22a ahead against the contact pad 10a. Due to the fact that the contact portion 22 is movable normal to the components 10, 11 (i.e. along virtual axis z), the contact device 2 can compensate tolerances in the distance D between the components 10, 11.

As further shown in Fig. 1 (A) and (B), the at least one first spring 21 mayy be wound about said central axis z of the connection device 2, wherein the contact portion 22 and the base portion 20 face one another in the direction defined by said central axis z. Particularly, the connection device 2 may comprises a second spring (not shown) for pressing the front side 22a of the contact portion 22 against the contact surface 10a, wherein the second spring may either connect (particularly integrally) the contact portion 22 to the base portion 20, or may alternatively be only connected to the base portion 20 and butt against a back side 22b of the contact portion 22. Also, the second spring can be wound around said virtual central axis z.

Particularly, in a preferred embodiment, the connection device 2 may be characterized by dimension s being smaller or equal to about 1.4mm x 1.4mm x 0.7mm. In another embodiment, the connection device may be characterized by dimension being smaller or equal to about 1.4mm x 1.4mm x 2mm.

Preferably, according to embodiments, the spring contacting provided by the contact portion 22 is used e.g., in the internal structure of implantable medical devices 1 such as cardiac pacemakers, implantable cardioverter defibrillators, cardiac monitors, or implantable leadless pacemakers.

Particularly, the connection device (or multiple such devices) 2 may be used to wire components such as header, feedthrough, battery, capacitor, electronic modules of implantable medical devices without internal wiring strips as well as welded connections. Furthermore, the contact portions 22 may also be used for contacting plugs of electrode leads in the header area of neurostimulators as well as implantable cardiac pacemakers and implantable cardioverter defibrillators. The contact portions 22 may also be used to replace a welded connection in the transition area between a header and a feedthrough of an implantable medical device, which would allow a plug-in mounting of the header.

Particularly, in the context of Fig. 1 (B), according to an embodiment, the first component 10 may be a component of an implantable medical device 1 such as an electronic module 101, wherein the second component 11 may be a feedthrough providing an electrical connection to a header, shown in detail in Fig. 2. The medical device comprise an electrically conductive housing 120, an electronic module 101 and an electrical feedthrough 112 having an insulator 112, one ore more feedthrough conductors extending through the insulator 113, and flange 114 surrounding the insulator 112, wherein the flange 114 is configured to be joined, particularly welded, to the electrically conductive housing 120. Here, one or more connection devices 2 according to the invention are arranged on the electronic module 101 and configured to establish an electrically conductive connection between the electronic module 101 and the electrical feedthrough 111, or more precisely to the one ore more feedthrough conductors 113

According to yet another embodiment, the first component 10 may be an electronic module 101 of an implantable medical device, wherein the second component 11 may be any component connected to the electronic module (such as a battery or a connector). Here, the contact portion may also connect to the second component 11. Fig.3 shows in detail a respective embodiment, in which the first component 10 is designed in form an electronic module 101 and the second component 11 in form of a battery 114. Here, two connection devices 2, 2a, 2b are arranged on and attached to a battery, wherein preferably one connector device 2a is in electrically conductive connection to the positive terminal of the battery 155, and the other connector device 2b to the negative terminal of the battery 115. The electronic module 101 in this embodiment comprises two electrically conductive contact member 103, which are configured as limit stop elements for the connector devices 2, 2a, 2b on the battery 114, wherein the contact members are preferably soldered to respective pads 102 on the electronic module 101. As can be seen in Fig. 3, the electronic module 101 with the connector elements 103 and the battery 115 with the connector devices 2a, 2b are arranged to each other such, the connector device can abut to the contact members 103 in order to provide an electrical connection, respectively, However, it is also conceivable that the connector devices 2 according the invention are arranged on and attached to the electronic module 101 such that the connector devices 2 can abut to positive and negative terminal of the battery 115 in order to establish an electrical connection, respectively.

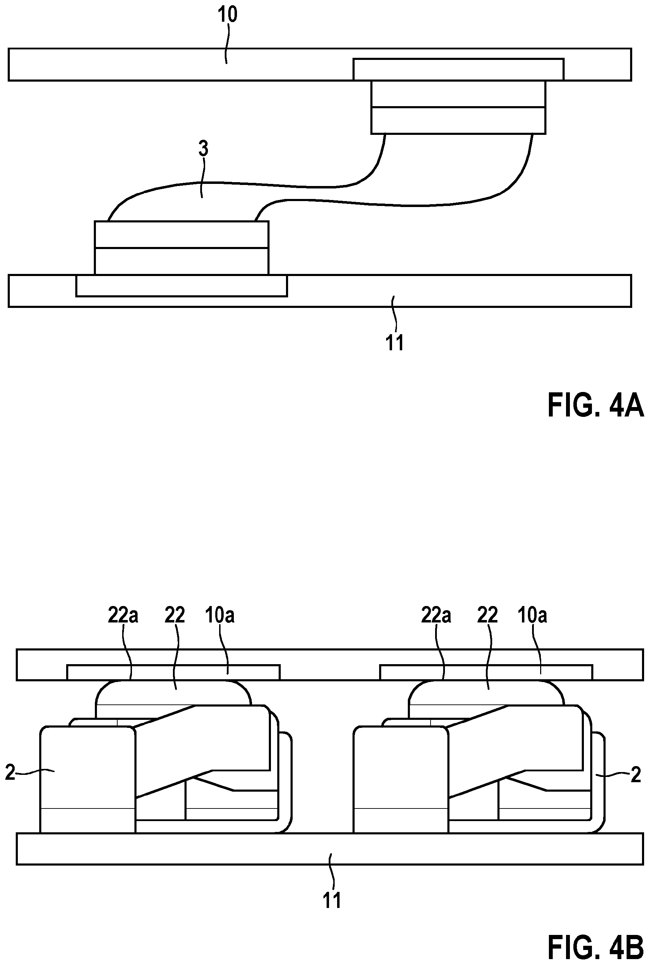

Furthermore, as shown in Figs. 4 (A) and (B) the tensioned contact portions 22 of the connection device 2 may also replace a flex cable connector 3 connecting two components / printed circuit boards 10, 11 of a medical device, particularly implantable medical device.

The present invention offers the advantages that, with the same size, electrical contacting by pick and place robots would be possible while also ensuring compensation of tolerances of individual components. Particularly, clamping and securing by snap clamps as with FPC connections can be eliminated. Likewise, welding of components (HSK, DF, battery and capacitor) in the internal structure of CRM products using internal wiring tapes can be avoided as well using the present invention.

## Claims

1. A medical device (1), comprising:
- a first component (10, 101, 115) comprising an electrically conducting contact surface (10a),
- a second component (11, 101, 111, 115) having an electrically conducting contact member (11a, 112, 103), and
- an electrically conducting connection device (2) electrically connecting the contact surface (10a) of the first component (10) to the contact member (11a) of the second component (11),
**characterized in that**
the connection device (2) comprises a base portion (20) electrically connected to said contact member (11), a contact portion (22), and at least one spring (21) electrically connecting the base portion (20) to the contact portion (22), wherein the at least one spring (21) is tensioned and thereby presses a front side (22a) of the contact portion (22) against the contact surface (10a) so that the contact portion (22) permanently contacts the contact surface (10a).

2. The medical device according to claim 1, wherein the at least one spring (21) is wound around a virtual central axis (z) extending orthogonal to the front side (22a) of the contact portion (22), wherein the contact portion (22) faces the base portion (20) in a direction defined by the central axis (z).

3. The medical device according to claim 2, wherein the spring (21) is compressed in said direction and thereby tensioned.

4. The medical device according to one of the preceding claims, wherein the spring (21) integrally connects the contact portion (22) to the base portion (22).

5. The medical device according to one of the preceding claims, wherein the base portion (20) is connected to the contact member (11a) by one of: a welded connection, a soldered connection, an electrically conductive adhesive connection, a crimp connection.

6. The medical device according to one of the preceding claims, wherein the front side (22a) comprises a planar region contacting the contact surface (10a), wherein the planar region comprises an area in the range from 0.01 mm² to 1.5 mm², preferably 0.1 mm² to 1 mm², most preferably 0.1 mm² to 0.5 mm².

7. The medical device according to one of the preceding claims, wherein the front side (22a) comprises a contact nose (22c) for contacting the contact surface (10a), wherein the contact nose (22c) is elastically supported on the contact portion (22) so that the contact nose (22c) is flush with a surrounding region of the front side (22a), when the front side (22a) is pressed against the contact surface (10a) of the first component (10).

8. The medical device according to one of the preceding claims, wherein the connection device (2) comprises a second spring for pressing the front side (22a) of the contact portion (22) against the contact surface (10a) of the first component (10).

9. The medical device according to claim 8, wherein the second spring connects the contact portion (22) to the base portion (20), or wherein the second spring butts against a back side (22b) of the contact portion (22) and is connected to the base portion (20), wherein said back side (22b) faces away from said front side (22a).

10. The medical device according to claim 2 and according to claim 8 or 9, wherein the second spring is wound around said central axis (z).

11. The medical device according to one of the preceding claims, wherein the first component (10) is an electronic module (101) and wherein the second component is versa battery (115) or vice versa.

12. The medical device according to one of the claims 1 to 10, wherein the first component (10) is an electronic module (101), and wherein the second component (11) is an electrical feedthrough (111) or vice versa.

13. The medical device according to one of the claims 1 to 10, wherein the first component (10) is an electrode lead and the contact surface (10a) is formed by a plug of the electrode lead, and wherein the second component (11) is a header comprising a socket for receiving said plug.

14. The medical device according to one of the preceding claims, wherein the medical device is an implantable medical device (1).

15. The medical device according to claim 14, wherein the implantable medical device (1) is one of: an implantable cardiac pacemaker, an implantable cardioverter defibrillator, an implantable cardiac monitor, an implantable leadless pacemaker, a neurostimulator, a vagus nerve stimulator.
